# EUROPEAN PATENT SPECIFICATION

(11) **EP 0 579 607 B1**
(45) Date of publication and mention of the grant of the patent: **13.03.1996**
(21) Application number: 92902434.7
(22) Date of filing: 13.01.1992
(51) Int. Cl.: G01N 33/574

(54) **PRESCREENING OF PROSTATE CANCER BY SERUM PROSTATIC SPECIFIC ANTIGEN**
VORPRÜFUNG VON PROSTATAKREBS DURCH SERUM PROSTATISCHEN SPEZIFISCHEN ANTIGEN
PREDEPISTAGE DU CANCER DE LA PROSTATE GRACE A UN ANTIGENE SPECIFIQUE DE LA PROSTATE PRESENT DANS LE SERUM

(30) Priority: 14.01.1991 US 640996
(43) Date of publication of application: 26.01.1994
(73) Proprietor: ENDORECHERCHE INC., Ste-Foy Quebec G1W 2J6 (CA)
(72) Inventor: LABRIE, Fernand, Quebec, Quebec G1W 2J6 (CA)
(74) Representative: Dauster, Hanjörg, Dipl.-Ing.
(86) International application number: CA9200015
(87) International publication number: WO9212430

(56) References cited:
- MONOGRAPHS IN UROLOGY, vol. 10, 1989; T.A. STAMEY, pp. 50-64/
- JOURNAL OF UROLOGY, vol. 142, no. 4, October 1989; M.A. HUDSON et al., pp. 1011-1017/
- UROLOGY, vol. 28, no. 6, December 1986; B. SEAMONDS et al., pp. 472-479/

## Description

### BACKGROUND OF THE INVENTION

This invention relates to a method using serum prostatic specific antigen (PSA) levels in the general population, preferably of men over 45 years of age, to indicate whether increased risk of possible prostate cancer justifies more elaborate diagnostic procedures in order to determine the presence or absence of prostate cancer. The invention further relates to kits for carrying out the method.

Based on the finding of a high incidence of prostate cancer at autopsy in men more than 50 years old (Lundberg et al., Scand. J. Urol. Nephrol. 4: 93-97, 1970), prostate cancer has generally been considered as a relatively benign disease or a normal and irrevocable component of the aging process. There is, however, ample evidence to change this concept. In the United States alone, it was predicted that 109,000 new cases of prostate cancer would be discovered in 1990, thus making prostate cancer the commonest cancer in men (Silverberg et al., CA-A Cancer J for Clinicians 40: 9-28, 1990). Moreover, as the population ages, the impact of prostate cancer will continue to increase (Chisholm, Rec. Res. Cancer Res. 78: 173, 1981). Already, prostate cancer is the second cause of cancer death in men in North America (Silverberg et al., CA-A Cancer J. for Clinicians 40: 9-28, 1990).

Prostatic carcinoma is found in 15 to 26% of prostatectomy specimens after resection for benign prostatic hyperplasia by routine sectioning. However, the incidence rate increases to 25 to 40% if step sections are used (Joslin et al., South Med. J. 54: 233-237, 1961; Denton et al., J. Urol. 93: 296-298, 1965). At autopsy, a similar incidence is found, namely 15 to 17% by random sections and 46 to 57% by step sections (Baron and Angrist, Arch. Pathol. 32: 793-798, 1941). The life-time probability of diagnosis of this disease is 5.2% and 9.6% in white and black males, respectively (Mettlin and Natarajan, Prostate 4: 323-331, 1983). In addition to the high frequency of prostate cancer diagnosed in 1 out of 20 white North American males during their lifetime, more than 50% of the patients have reached the stage of bone metastases (stage D2) when the cancer is first discovered. At this late stage, endocrine therapy is the only efficient treatment available, and, despite its recent improvement (Labrie et al., Clin. Invest. Med. 5: 267-275, 1982), this treatment is only palliative (Jordan et al., South Med. J. 70: 1411-1413, 1977). (See generally, Labrie et al., In: Genitourinary Cancer (Catalona WJ, Ratliff TL, eds), Boston: Martinus Nijhoff Publ., pp. 157-200, 1987).

In fact, a major problem facing the treatment of advanced prostate cancer is the development of resistance to endocrine therapy, thus leaving no effective alternative treatment (Labrie et al., In: Genitourinary Cancer (Catalona WJ, Ratliff TL, eds), Boston: Martinus Nijhoff Publ., pp. 157- 200, 1987; Labrie et al., J. Urol. 138: 804-806, 1987b; Labrie et al., Brit. J. Urol. 61: 341-346, 1988). Chemotherapy, on the other hand, has given disappointing results with the drugs presently available (Eisenberger et al., Urol. Clin. North Amer. 14: 695-706, 1987).

The main reasons which can explain the usual late diagnosis of prostate cancer is the absence of pain or other symptoms during the many years of progression of the tumor in the prostate itself, its surrounding tissues, the lymph nodes and even in the bones. Usually, pain appears only after extensive spread into the bones. Moreover, the laboratory tests so far available could not be used with sufficient specificity and sensitivity to detect localized prostate cancer.

It is believed that earlier diagnosis at a time when cancer is still limited to the prostate could allow use of curative techniques which are unavailing at later stages of the diseases. This should most likely improve survival rates for prostate cancer patients. Somewhat surprisingy , despite the fact that early treatment potentially offers a curative therapy, relatively little attention has so far been paid to the diagnosis and treatment of early stage prostate cancer (Editorial: Prostate Cancer Consensus Hampered by Lack of Data, Science 236: 1626-1627, 1987).

The advantages of early treatment are supported by the recent data indicating that an increase in tumor volume is associated with a higher incidence of poorly differentiated cancer (Stamey et al., J. Urol. 139: 1235-1241, 1988). In fact, the recent study of Stamey et al. (J. Urol. 139:1235-1241, 1988) indicates that tumors having a volume greater than 4.0 cc have a high likelihood of metastases while tumors smaller than 4.0 cc have a much higher potential for surgical cure. Moreover, surgical and autopsy studies have shown that smaller lesions are much less often associated with penetration of the capsule and are thus potentially curable (Scott et al., 1969; J. Urol. 101: 602-607, 1969; McNeal, Cancer 23: 24-34, 1969; Culp and Meyer, Cancer 32: 1113-1118, 1973; McNeal et al., The Lancet ii: 60-63, 1986). In addition, more advanced tumor stage is accompanied by a higher local recurrence rate after radiation therapy and by a higher incidence of distant metastases (Barzell et al., J. Urol118: 278-282, 1977; Cupps et al., J. Urol. 124: 855-859, 1980; Grayhack and Assimos, Prostate 4: 13-31, 1983; Kuban et al., Urology 30: 420-426, 1987). As mentioned above, it is thus recognized that the invasiveness of cancer and its dissemination to periprostatic tissues, lymph nodes and bones are proportional to cancer volume and to the percentage of poorly differentiated areas.

The significance of histologic grade of prostatic cancer as prognostic factor of the evolution of the disease is also well known (Gleason and Mellinger, J. Urol. Ill: 58-64, 1974; Grayhack and Assimos, Prostate 4: 13-31, 1983). This is particularly well illustrated by the stepwise increase in disease metastases from well differentiated (10%) to moderately differentiated (30%) and to poorly differentiated carcinoma (48%) after 3 years of follow-up (Kuban et al., Urology 30: 420-426, 1987 A ). progressive increase in the incidence of metastases is thus seen with loss of histologic differentiation (Kuban et al., Urology 30: 420-426, 1987), which, in turn, is proportional to tumor volume (Stamey et al., J. Urol. 139: 1235-1241, 1988). Smaller tumors are thus associated with better histological grade and lower cancer aggressiveness.

So far, the standard method for early diagnosis of prostate cancer has been digital rectal examination (Guinan et al., New Engl. J. Med. 303: 499-503, 1980). This procedure, although being the standard technique for evaluation of tumor size, prognosis and response to therapy (Stamey, Monogr. Urol. 4: 68-72, 1983), is relatively inaccurate, especially for small tumors, which can often be underestimated or completely missed (Fair and Kadmon, World J. Urol. 1: 3-11, 1983; Spigelman et al., J. Urol. 136: 1228-1230, 1986; Lee et al. , In: Early Stage Prostate Cancer: Diagnosis and Choice of Therapy (Labrie, F. , Lee, F. , Dupont, A. , eds) , Excerpta Medica ICS 841: 23-26, 1989). Understaging has been estimated to occur in up to 72% of patients with stage B disease (Fair and Kadmon, World J. Urol. 1:3-11, 1983). In other studies, the specificity of the rectal examination has been reported to range from 29 to 45% (Jenson et al., JAMA 174:1783- 1788, 1960; Chodak and Schoenberg, JAMA 252: 3261-3264, 1984).

For example, in the study of Chodak and Schoenberg, JAMA 252: 3261- 3264 (1984) performed in 811 men, 656 being aged between 51 and 80 years, 43 were found to have prostate abnormalities suggesting carcinoma by digital rectal examination. Eleven of these 43 men were identified as having prostate cancer by biopsy. The specificity of the rectal examination was thus 29% (11/38) and the overall incidence of prostatic carcinoma in this high risk age group (51 to 80 years) was 1.7% (11/656). In the study of Gilbertsen (JAMA 215: 81-84, (1971), where digital examination was performed once a year for five years on 5856 men, 75 prostatic carcinoma were detected (1.3%).

It is of special interest to mention that in another study (Jenson et al., JAMA 174: 1783-1788, 1960), it was found that survival was improved in patients who were diagnosed as having prostate cancer during their second screening examination compared to those who had diagnosis at the first visit. Since larger tumors were likely to have been diagnosed in the first year of screening, such data indicate the advantage of detection and treatment of smaller tumors. The 5- and 10-year survivals in the group of patients who had radical prostatectomy performed for cure after detection of local disease showed that these patients had a life expectancy virtually identical to that of men of comparable age in the general population without disease (Jenson et al., JAMA 174: 1783-1788, 1960). Such data suggest that regular examination, even with the relatively insensitive technique of digital rectal examination, can detect patients with less extensive disease and permit early curative treatment and improve survival.

As mentioned above, until recently, digital rectal examination was the standard modality for detection of early stage prostate cancer (Guinan, P. et al. New Engl. J. Med. 303: 499, 1980) but its sensitivity is limited (Franks, J. Pathol. Bacterial. 68: 603, 1954; Stamey et al., J. Urol. 139: 1235, 1988). In fact, the number of detected prostate cancers can be approximately doubled using high frequency transrectal ultrasonography for screening (Lee et al., Radiology 170: 609-615, 1989).

In fact, during the last few years, the development of high frequency transducers has literally brought transrectal ultrasonography from a relatively crude and imprecise technique to one which permits, with an unprecedented accuracy, evaluation of the anatomy and the pathological status of the prostate. It now permits detection of small size tumors as well as an accurate estimate of tumor volume (Lee et al., The Prostate 7: 117-129, 1985; Dahnert et al., Radiology 158: 97-102, 1986; Lee et al., Radiographics 7: 627-644, 1987; Radiology 163: 515-520, 1987; In: Early Stage Prostate Cancer: Diagnosis and Choice of Therapy (Labrie, F., Lee, F., Dupont, A., eds), Excerpta Medica ICS 841, 23-36, 1989).

The higher sensitivity of transrectal ultrasonography compared with digital rectal examination has recently been demonstrated in a screening study performed in 388 men (Lee et al., Radiographics 7: 627-644, 1987). Thirteen cancers were detected for an incidence of 3.3%. Of the positive biopsies, 92% (12/13) were detected by transrectal ultrasonography and only 54% (7/13) by digital rectal examination. Of the tumors measuring less than 1.5 cm by ultrasonography, 45% were not palpable. One conclusion of the authors was that: "transrectal sonography has the ability to detect non palpable, potentially curable cancer and holds great promise as a screening modality".

Such data clearly suggest the importance of transrectal prostatic ultrasonography in the diagnosis of prostate cancer at the preclinical phase since two potentially curative approaches can be offered to the patients, namely nerve sparing radical prostatectomy (Walsh et al., The Prostate 4: 473- 485, 1983) and high voltage radiotherapy.

Prostatic specific antigen (PSA) is synthesized exclusively by normal prostatic epithelial cells as well as by neoplastic cells in tumors localized within the prostate or metastases of prostatic cancer at extraprostatic and distant sites (Pontes et al., J. Urol. 128: 1216, 1982; Allhoff et al., J. Urol. 129: 315, 1983; Killian et al., Cancer Res. 45: 886, 1985; J. Natl Cancer Inst. 76: 179, 1986; Hammond et al. 63: 461, 1989) .

PSA was fully characterized following purification from semen (Sensabaugh, J. Forensic. Sci. 23: 106, 1978) and prostatic tissue (Wanq et al. Invest. Urol. 17: 159, 1979) . This protein has not been found in any normal or tumor cells other than prostatic tissue (Nadji et al. , Cancer 48: 1229, 1981; Kuriyama et al. , Cancer Res. 40: 4658, 1980).

Since PSA is also present in normal prostatic tissues, thus contributing to an unknown extent to serum PSA levels, single measurement of serum PSA as a specific marker as useful for screening and diagnosis of prostate cancer could not be recommended (Pontes et al., J. J. Urol. 128: 1216, 1982; Myrtle et al., Adv. Cancer Diagnostics, pp. 1-6, 1986; Seamonds et al., Urology 28: 472, 1986; Armitage et al., Brit J. 62: 472, 1986; Killian et al., J. Natl Cancer Inst. 76: 179, 1986; Guinan et al., J. Urol. 137: 686, 1987; Ahmann and Schifman, J. Urol.137: 431, 1987; Ercole et al., J. Urol. 138: 1181, 1987; Stamey et al., New Engl. J. Med. 317: 907, 1987).

Benign prostatic hyperplasia (BPH) elevates serum PSA levels at a rate of 0.3 ng/ml per gram of BPH tissue (Stamey et al., New Engl. J. Med. 317: 909, 1987; Stamey, J. Urol. 141: 1076, 1989) while prostate cancer has been estimated to elevate serum PSA at an approximately 10- fold greater rate, namely 3.5 ng/ml per gram of cancer cells (Stamey et al., J. Urol. 141: 1076, 1989) as determined by the Yang polyclonal radioimmunoassay. Hudson et al. J. Urol, Vol 142, p1011 determine PSA levels for various populations of patients, before and after treatment with ot without confirmed prosate cancer, etc.

So-far, most of the PSA studies have used one of the two commercially available radioimmunoassays, with variable normal values (Pontes et al. , J. Urol. 128: 1216, 1982; Killian et al. , Cancer Res. 45: 886, 1985; Seamonds et al. , Urology 28: 472, 1986; Armitage et al. , Brit. J. Urol. 62: 472, 1986; Killian et al. , J. Natl Cancer Inst. 76: 179, 1986; Myrttle et al., Adv. Cancer Diagnost, pp. 1-16, 1986; Ahmann and Schifman, J. Urol. 137: 431, 1987; Ercole et al., J. Urol. 138: 1181, 1987; Guinan et al., J. Urol. 137: 686, 1987; Stamey et al., New Engl. J. Med. 317: 909, 1987). With some variations, especially at both ends of the standard curve, the Yang assay reads, for a given sample, about 1.65 times higher than the Hybritech assay (Stamey, Monogr. Urol. 10: 50-64, 1989).

As mentioned earlier, tumor volume appears to be the best indicator of the stage of evolution of the disease since capsular penetration, seminal vesicle and lymph node invasion as well as lymph node and bone metastases tend to increase as tumor volume increases (McNeal et al., The Lancet i: 60-63, 1986). Unfortunately, direct assessment of tumor volume by transrectal ultrasonography is not practical for individual men in the general population. The large number of radiologists and/or urologists required and the costs involved make such an approach impossible to generalize. There is thus an urgent need to develop a test with high sensitivity and specificity which is quick, inexpensive, minimally invasive and acceptable to the general population of men without symptoms.

The only data available so far on the correlation between serum PSA values and prostatic tumor volume are those obtained following radical prostatectomy. Although a strong correlation is always found between serum PSA levels and tumor volume (Stamey et al., New Engl. J. Med. 317: 900-916, 1987; Stamey et al. J. Urol. 141: 1076-1083, 1989; Brawer et al. , J. Endocrinol. 3: 227-236, 1989) , it is most likely that the patients treated by radical prostatectomy in non-randomized studies and diagnosed following standard diagnostic procedures have tumors of larger volume and thus higher PSA values than those of the general population of men without symptoms.

Various cut-of f serum PSA values have been suggested but none were based on studies performed in the asymptomatic general population chosen at random in order to eliminate bias caused, for example, by more frequent consultation by men having a familial history of prostate cancer or urogenital symptomatology. Thus, Lee et al. used a cut-off value of 2.6 ng/ml for the Yang assay which is equivalent to about 1.4 ng/ml for the Hybritech test (Lee et al., 5th Int. Symp. Transrectal Ultrasound in the Diagnosis & Management of Prostate Cancer, Chicago, pp. 125-126, 1990).

Determining the normal range for plasma PSA is problematic, especially due to the high prevalence of BPH in men. The manufacturer's suggested normal values of the Hybritech immunoradiometric assay (Tandem-R PSA) range up to 4. 0 ng/ml. The same value has been used by Escole et al. (J. Urol. 138: 1181-1184, 1987) and Oesterling et al. (J. Urol. 139: 766-772, 1988). With a value of 4.0 ng/ml, 22% (4 of 18) of patients with seminal vesicle invasion had PSA levels within the normal range (Oesterling et al., 1988). An upper normal value of 4.0 ng/ml (Hybritech) has also been chosen by Cooner et al. (5th Int. Symposium on Transrectal Ultrasound in the Diagnosis & Management of Prostate Cancer, Chicago, 1990).

With the Yang assay, Stamey et al. used 2.5 ng PSA/ml as the upper limit of normal, this value corresponding to approximately 1.5 ng/ml in the Hybritech assay (Stamey et al., J. Urol. 141: 1076, 1989). Lange et al. (J. Urol. 141: 873-879, 1989), on the other hand, used 10 ng/ml as the upper limit (Hybritech assay).

In a relatively small group of patients undergoing simple prostatectomy for bladder outlet obstructive symptoms related to presumed BPH, using a cut-off of 4.0 ng/ml with the Tandem-R PSA assay, 71% of patients with adenocarcinoma had elevated values while only 4% having exclusively BPH had elevated serum PSA (Brawer et al. , kn. J. Clin. Pathol., in press). When analyzing the serum PSA values of series of patients undergoing radical prostatectomy and using a cut-off of 2.8 ng/ml with the Tandem-R assay (Hybritech) , 71 to 89% of patients had an abnormal PSA while with a cut-off of 4.0 ng/ml, 29 to 78% of patients had abnormal PSA in various studies (Lange et al. , NCI Monograms 7: 141-149, 1988; Partin et al., J. Urol. , in press; Hudson et al. , J. Urol. , in press; Brawer and Lange, J. Endocrinol. 3: 227-236, 1989).

Despite these previous difficulties in determining normal serum PSA values, a close correlation is recognized between prostatic cancer volume and serum PSA values (Stamey et al., New Engl. J. Med. 317: 909, 1987; Brawer and Lange, J. Endocrinol. 3: 227, 1989; Stamey et al., J. Urol. 141: 1076, 1989). Moreover, high serum PSA values are indicative of advancing clinical stage of the disease (Killian et al., Cancer Res. 45: 886, 1985; Seamonds et al., Urology 28: 472, 1986; Myrtle et al., Adv. Cancer Diagnostics, pp. 1-6, 1986; Ercole et al., J. Urol. 138: 1181, 1987; Stamey et al., New Engl. J. Med. 317: 909, 1987).

In a small series of patients who underwent simple prostatectomy, the specificity of PSA (Hybritech) was 62.7% using a cut-off of 4.0 ng/ml (Lange et al., NCI Monograph 7: 141-149, 1988) while in a series of transrectal ultrasonography guided needle biopsies, a specificity of 60.3% was noted at 4.0 ng/ml (Bostwide et al., Cancer 59: 788-784, 1987). Cooner et al. (5th Int. Symposium on Transrectal Ultrasound in the Diagnosis and Management of Prostate Cancer, Chicago, pp. 71-73, 1990) noted a specificity of 61.3% in a similar series of transrectal ultrasonography guided needle biopsies.

The best recognized utility for PSA today is the monitoring of patients with established carcinoma and especially following radical prostatectomy. Following successful surgical ablation of the prostate tumor(s), PSA levels should fall into the undetectable range. Persistence of measurable PSA following radical prostatectomy has been correlated with more advanced disease. An elevation of serum PSA following surgery indicates recurrence of tumors (Stamey et al., New Engl. J. Med. 317: 909, 1987; Chan et al., Clin. Chem. 33: 1916, 1987; Oesterling et al., J. Urol. 139: 766-772, 1988; Lange et al., NCI Monogr 7: 141-149, 1988; Stamey et al., J. Ural. 141: 1076-1083, 1989; Hudson et al., J. Urol. 142: 1011-1017, 1989).

Stamey et al. (J. Urol. 141: 1076, 1989) have found serum PSA levels above 0.3 ng/ml in 15 of 97 (15%) patients during the first 12 months following radical prostatectomy. Using a value of 0.2 ng/ml, Lange et al. (J. Urol. 141: 873, 1989) have found elevated PSA values in 23 of 59 (38.9%) men 3 to 6 months after surgery. When a value of 0.6 ng/ml was used, 14 of 93 (15%) patients had elevated serum PSA levels measured within 6 months after surgery.

Similarly, rising serum PSA levels following radiotherapy or endocrine therapy indicate recurrence of the disease (Killian et al., J. Natl Cancer Inst. 76: 179, 1985; Labrie et al., In: Important Advances in Oncology (De vita VT, Hellman S, Rosenberg SA, eds), Philadelphia: J.B. Lippincott, pp. 193-217, 1985; Seamonds et al., Urology 28: 472, 1986; Killian et al., Cancer Res. 45: 886, 1986; Myrtle et al., Adv. Cancer Diagnostics, pp. 1-6, 1986; Ercole et al. , J. Urol. 138: 1181, 1987; Stamey et al. , New Engl. J. Med. 317: 909, 1987; Labrie et al. , In: Genitourinary Cancer (Catalona WJ, Ratliff TL, eds) , Boston: Martinus Nijhof f Publ. , pp. 157-200, 1987; Labrie et al. , Eur. J. Cancer Clin. Oncol. 24: 1869, 1988; Labrie et al. , Brit. J. Urol. 61: 341-346, 1988).

The Gleason grade, extent of capsular penetration tion, seminal vesicle invasion and lymph node metastases all appear to be related to increasing cancer volume (Stamey et al., J. Urol. 141: 1070, 1989; Stamey et al., J. Urol. 139: 1235, 1988; McNeal et al., Lancet 1: 60, 1986). Although it is recognized that serum PSA levels in the untreated patient tend to vary with volume of prostate cancer, uncertainty about the contribution of nomal and BPH tissue to total serum PSA levels prevented the use of serum PSA for screening prostate cancer. Consequently, a highly sensitive yet reasonably specific serum PSA test is not presently known in the art for detection of cancers having a significant volume before their dissemination outside the prostatic capsule and at distant metastatic sites.

Despite the importance of early detection of prostate cancer, the major problem arising with the combined use of digital rectal examination, transrectal ultrasonography and PSA concentration for screening of prostatic cancer is the high costs involved, especially for transrectal ultrasonography, thus making mass screening in the general population too complex and expensive.

All previous studies of the correlation between serum PSA levels and screening or staging of prostate cancer have been performed in patients already diagnosed as having prostate cancer (Oesterling et al., J. Urol. 139: 766-772, 1988; Stamey et al. , J. Urol. 141: 1076-1083, 1989; Lange et al., J. Urol. 141: 873-879, 1989) or upon those consulting medical personnel because of symptoms or suspicion of prostate cancer or susceptibility.

As support for early treatment of prostate cancer, recent advances in the surgery of prostatic carcinoma should significantly improve the prognosis following radical prostatectomy. These modifications take into account: a) knowledge of the sites of penetration of cancer into the periprostatic fat (McNeal et al., Lancet i: 60, 1986; Stamey et al., J. Urol. 139: 1235, 1988; Villers et al. , J. Urol. 142: 763, 1989); b) identification of the areas of invasion of the Denonvilliers' fascia (Villers et al. , J. Urol. 143: 1183-1187, 1990; c) detailed analysis of the positive margins (Stamey et al., J. Urol. 143: 1183-1187, 1990) and d) knowledge of the route of spreading of the cancer along the ejaculatory ducts into the seminal vesicles (Villers et al., J. Urol. 143: 1183-1187, 1990).

Already, it has been suggested, mainly based on digital rectal examination that screening programs are cost effective, identify a high proportion of patients with early disease and are likely to prolong survival (Love et al. , Med. Decis. Making 5: 263-278, 1985; Thompson et al. , J. Urol. 137: 424-426, 1987; Imai et al. , Prostate 12: 199-1207, 1988; Chadwick et al., Brit. Med. J., 301: 119-120, 1990).

In previous evaluations of screening programs, populations were not usually randomly selected (Chodak and Schoenberg, JAIIA 252: 3261-3264, 1984; Vihko et al., Cancer 56: 173-177, 1985; Lee et al., Radiology 168: 389-394, 1988). Digital rectal examination was used purportedly with a random population by Pedersen et al. (Brit. Med. J. 300: 1041-1044, 1990). However, low sensitivity for digital rectal examination has been recognized (Thompson et al., J. Urol. 132: 690-692, 1984; Vihko et al., Cancer 56: 173-177, 1985). In fact, cancer was found in only 13 of 1162 (1. 1%) men aged 50 to 69 years examined, a value much lower than that reported when transrectal ultrasonography is used (Lee et al. , Radiology 170: 609-610, 1989).

The presently available techniques, namely radical prostatectomy and radiation therapy can theoretically cure the disease and have in fact been shown to have excellent 10- and 15-year survival rates when early stage cancer is treated (Paulson et al. , J. Urol. 128: 502, 1982; Bagshaw, Urol. Clin. N. Amer. 11: 297, 1984). Thus, if all cases of prostate cancer are diagnosed and treated at an early stage, the number of men suffering from metastatic and non curable prostate cancer should be minimal, and death rates should fall appreciably.

In the prior art, no appropriate positive indication threshold value for serum PSA had been derived from random sampling. In the absence of selection of an appropriate threshold value, too many cancers could be left undetected in a screening test, or too many false positive indications might result. In the absence of a threshold value determined in a randomly selected population of asymptomatic men, serum PSA was not judged recommendable for screening of prostate cancer.

Other techniques are of limited value as prescreening techniques for the general population, or as techniques which may be performed in routine physical examinations. Digital rectal examination, for example, is too insensitive and time consuming. Transrectal ultrasonography is both too time consuming and expensive.

### SUMMARY OF THE INVENTION

It is an object of the present invention to provide a specific method of prescreening prostate cancer in the general population.

It is another object of the invention to provide an efficient method of screening of prostate cancer which will permit early diagnosis and treatment of prostate cancer at a time when a curative approach (e.g. radical prostatectomy or radiotherapy) can be offered to the patient, and to decrease the occurrence of advanced prostate cancer when only palliative therapies are available.

It is another object of the present invention to provide a cost-effective prescreening and diagnosis of prostate cancer.

It is another object of the present invention to provide a minimally invasive method of prescreening of prostate cancer which is rapid, practical for the general population, and requires minimal time from medical personnel.

These and other objects are achieved by providing the kits and methods disclosed herein for testing for the existence of prostate cancer.

In one embodiment of the invention, a method is provided for detection of possible prostate cancer comprising: Means for determining whether a sample of blood serum from a male person being tested includes a concentration of prostatic specific antigen which exceeds a predetermined threshold value; wherein said threshold value is between 2.0 and 3.9 nanograms of prostatic specific antigen per ml of said serum.

Concentration levels of prostatic specific antigen given herein are weight per volume concentrations of prostatic specific antigen in blood serum determined by using the "Hybritech Assay", which is explained in detail Tandem-RPSA, immunoradiometric assay published by Hybritech. Materials needed for performing the Hybritech Assay are 12sI-labeled monoclonal antibody against a specific site on PSA and plastic beads coated with antibody directed against a different site on the PSA molecule and may be obtained from Hybritech, 11095 Torreyana Road, P.O. Box 269006 (92126), San Diego, CA92121.

Naturally, the invention may be practiced using many other assays or techniques for measuring prostatic specific antigen concentration. Where other techniques are used to measure PSA concentration, appropriate conversions should be made before comparing an alternatively-measured concentration value to the Hybritech-measured threshold values used herein, thus accounting for inherent differences between concentration values obtained using different measuring techniques. For example, the "Yang Assay", which is fully described in Shaw et al., Clin. Chem. 27, 1505-1512, 1981) may be used to measure prostatic specific antigen concentration. Blood serum concentrations measured by the Yang Assay vary from the same concentrations measured by the Hybritech Assay in accordance with the following formula: ng PSA/ml in Yang assay ö - 1.65 = ng PSA/ml in Hybritech assay. The Yang assay for prostatic specific antigen concentration is the PROS-CHECK PSA RIA (Yang Laboratories, Bellevue, Washington).

In some methods of measuring concentration, whole blood may be used instead of serum. Naturally, appropriate conversions would be necessary to relate concentration numbers taken on serum to concentrations taken on whole blood. Likewise, if a blood or serum sample is concentrated or diluted prior to measurement of prostatic specific antigen levels, the dilution or concentration must be taken into account before comparison to the threshold values reported herein.

The present invention also includes a method for detecting possible prostate cancer in a male person, said method comprising the steps of:
(A) obtaining a blood or serum sample for said person; and
(B) determining whether a concentration of prostatic specific antigen in said sample exceeds a predetermined threshold value, said threshold value being between 2.0 and 3.9 nanograms of prostatic specific antigen per ml of serum. This method may be performed by using kits or devices in accordance with the present invention, or by sending serum samples to off-site laboratories for analysis of prostatic specific antigen concentration. The concentration reported by the testing laboratory is then compared to the threshold value to determine whether further testing to confirm prostate cancer is advisable. Because routine physical examinations include the taking of blood samples and transmitting such samples to a laboratory for analysis, the testing method of the present invention may be easily and inexpensively performed merely by including the present testing method together with other blood tests already being performed.

### BRIEF DESCRIPTION OF THE DRAWINGS

Figure 1 is a graph showing serum prostatic specific antigen concentrations distributed as a function of age in a randomized population (n=1002, r=0.184).

Figure 2 is a graph showing serum prostatic-specific antigen concentrations distributed as a function of age in 57 subjects diagnosed as having prostate cancer (r=0.398).

Figure 3 is a Receiver Operating Characteristic (ROC) curve calculated for prostatic-specific antigen. The area under the ROC curve, and its standard deviation are 87.8 +/- 3.3%.

### DESCRIPTION OF THE PREFERRED EMBODIMENTS

The method of the invention is particularly useful for the routine prescreening of the male population at large for possible prostate cancer, usually in connection with routine periodic physical examinations. While the tests described herein are believed to be most important with more susceptible males over 45 years of age, they are nonetheless recommended for the general population of such age. Such routine prescreening may increase the number of cancers diagnosed early enough for curative treatments to be applied.

In preferred embodiments, a blood sample is taken from a male person to be tested in a routine manner. The sample is then analyzed for concentration of prostatic specific antigen, either by sending the sample to an independent laboratory for testing, or by using a device or kit in accordance with the present invention. The device or kits may enable the user to determine a numerical concentration for prostatic specific antigen, or alternatively, may, upon use, merely indicate that the concentration is either above or below the predetermined threshold value. That threshold value should be set between 2.0 and 3.9 nanograms of prostatic specific antigen per ml of serum (Hybritech scale).

As shown below, a concentration of 3.0 nanograms per liter appears to give the best combination of sensitivity and specificity. Accordingly, a threshold value near that apparent optimum value, e.g., from about 2.5 to about 3.5 nanograms per ml is preferred. However, when the test is being used for a prescreening and one wants to further increase sensitivity, a lower threshold value of about 2.0 to 2.5 nanograms per ml is preferred. When a value between 2.0 and 2.5 nanograms per ml is chosen as the threshold value (upper limit above which the test is considered positive), it has been found that a lower percentage of prostate cancer-afflicted individuals would have blood concentrations of prostatic specific antigen falling in the "normal" range (i.e., below the threshold value). While specificity is sacrificed somewhat at this lower 2.0 to 2.5 nanograms per ml range, the test remains sufficiently predictive to be useful as a prescreen while further assuring that very few treatable cancers are missed.

A preferred kit for performing the method of the invention includes, for example, a means for taking and receiving a blood sample from a patient, and a means for determining the concentration of prostatic specific antigen in the sample. Preferred kits would include, for example, materials necessary for performing the Hybritech Assay, e.g., labelled anti-PSA monoclonal antibody and beads coated with another anti-PSA monoclonal antibody. A precise volume of serum (for example 0.10 to 0.50 ml) is added to the tube as well as some buffer to maintain optimal reaction conditions. Tests made with increasing amounts of purified PSA permit to calculate a standard curve and thus read the value of PSA present in the various serum samples. If materials for performing a different assay are included, a chart is preferably included for easy conversion of concentrations given by the assay of the kit to values determined by the Hybritech Assay. Indeed, where the kit includes materials for an assay other than Hybritech, a chart which directly sets forth the positive threshold value in the same units as the assay of the kit rather than in units of the Hybritech Assay may be preferable. In that event, no conversion is necessary prior to comparing the concentration derived from the assay to the threshold concentration which is to be considered a positive test result.

Testing devices in accordance with the invention may provide a numerical readout of blood or serum concentration of prostatic specific antigen which may then be compared to a chart for determining whether the concentration is sufficient to warrant further testing for prostate cancer, or whether the concentration is to be considered a negative test indication. Preferably, however, the device will be preset to perform the comparison automatically and to merely indicate the results (positive or negative) to the user. For example, the end result of analyzing a serum sample with such a device may be a color indication, one color indicating a positive result and another color indicating a negative result.

### SUBJECTS AND METHODS

Men aged between 45 and 80 years have been randomly selected from the electoral rolls of Quebec City and its vicinity. The selected men were invited to participate by letter and no announcement was made through public media in order to avoid bias. Subjects with prior diagnosis of prostate cancer were excluded from the study. One thousand and two randomly chosen men took part in the study.

In addition to answering a questionnaire on familial incidence of prostate cancer and providing information on genitourinary history and present symptomatology, digital rectal examination (DRE), transrectal ultrasonography of the prostate (TRUS) and serum prostatic specific antigen (PSA) measurements were performed. All men had signed an informed consent. Serum samples were obtained before DRE and TRUS for measurement of PSA by the iummunoradiometric assay (Tandem-R PSA, Hybritech, Inc). DRE was performed independently from the radiologist who was unaware of the digital rectal examination findings.

Transaxial and sagital scanning of the prostate were performed with a model 1846 Bruel & Kjaer apparatus using a 7.0 MH transducer (Lee et al., Radiology 168: 389-394, 1988). TRUS-guided biopsies were performed using the automatic Biopty System (Bard Urological Division, Covington, GA) with a 18-gauge needle. While we focused on hypoechoic lesions for biopsy, areas corresponding to a palpable abnormality by DRE were also biopsied. Moreover, random biopsies were performed in isoechoic areas of the peripheral and central zones. The pathological findings from random and TRUS-guided biopsies were the reference test for determining the presence or absence of prostate cancer. Subjects with negative DRE and negative TRUS were also considered as not having the disease.

Sensitivity, specificity, predictive values as well as likelihood ratios were calculated as described by Begg (Statistical methods in medical diagnosis. CRC Critical Reviews in Medical Informatics 1: 1-22, 1986). The area under the Receiver Operating Characteristic (ROC) curve has been calculated according to the method of Hanley and McNeil (Hanley and McNeil, Radiology 143: 29-33, 1982).

### RESULTS

Table 1 displays the distribution of serum PSA concentrations in relation with the presence or absence of detectable prostate cancer by digital rectal examination and transrectal ultrasonography confirmed by TRUSguined biopsy. It can be seen that when the upper limit of serum PSA is fixed at 20 ng/ml, 15 out of 17 (88,2%) men were found to have prostate

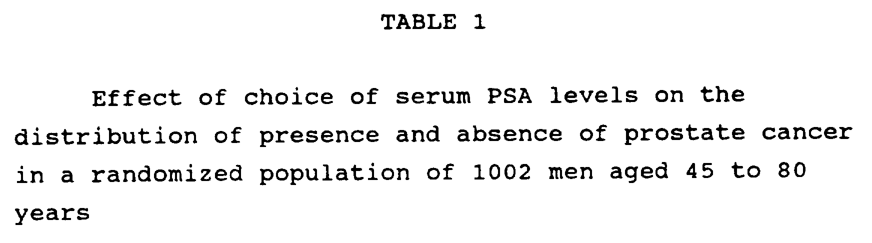

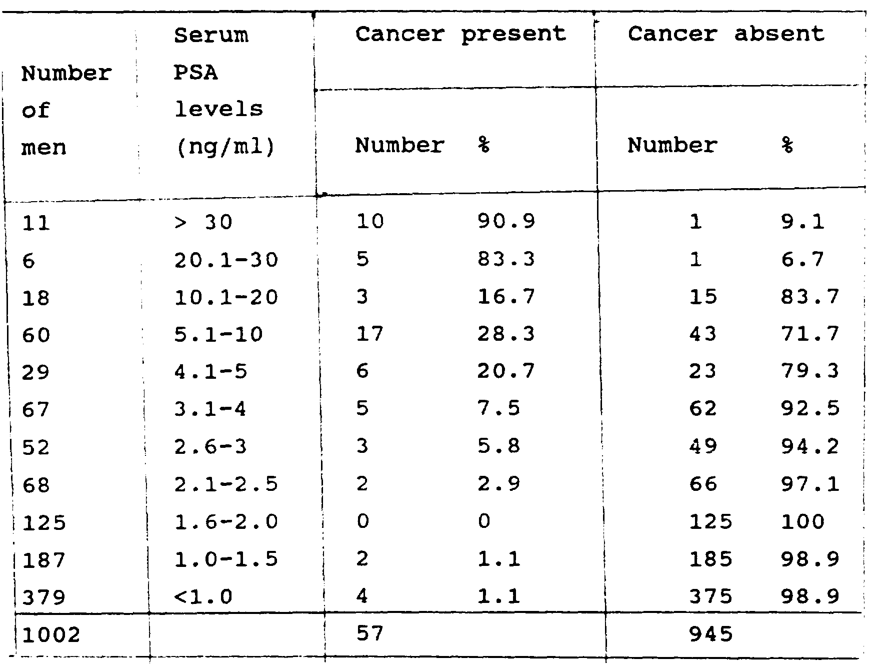

cancer. At the other extreme, however, when looking at normal serum PSA values up to 2.0 ng/ml, cancer was found at a 100-fold lower incidence rate, namely in only 6 out of 691 (0.87%) men, thus leaving 99. 1% of men with no detectable cancer. When the threshold PSA value was set at 3.0 ng/ml in order to increase the sensitivity of the assay, only 19.3% of cancers (11 out of 57) were missed by the prescreening PSA test while 84.6% of all men without cancer had serum PSA values up to 3.0 ng/ml or within the normal limits of the assay. As discussed later, those 11 men having cancer with serum PSA values up to 3.0 ng/ml are most likely to have small cancers which should become detectable by serum PSA measurements at later routine prescreening visits.

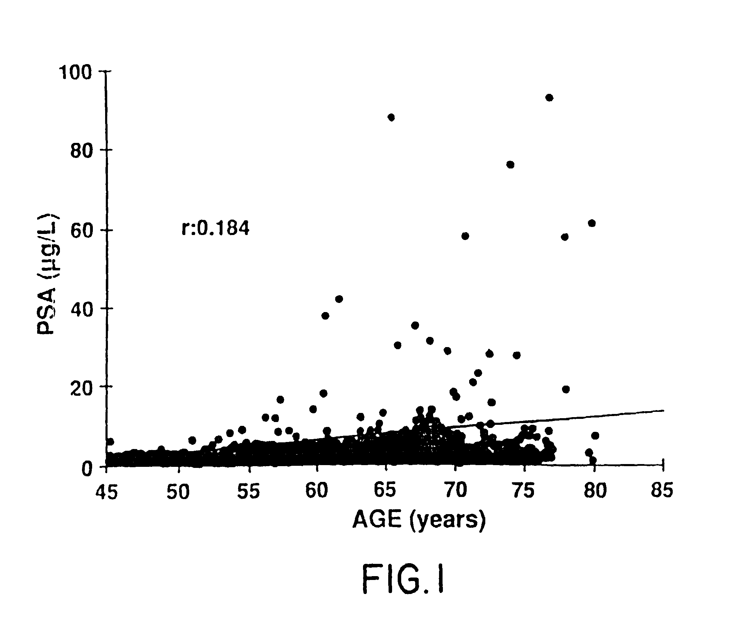

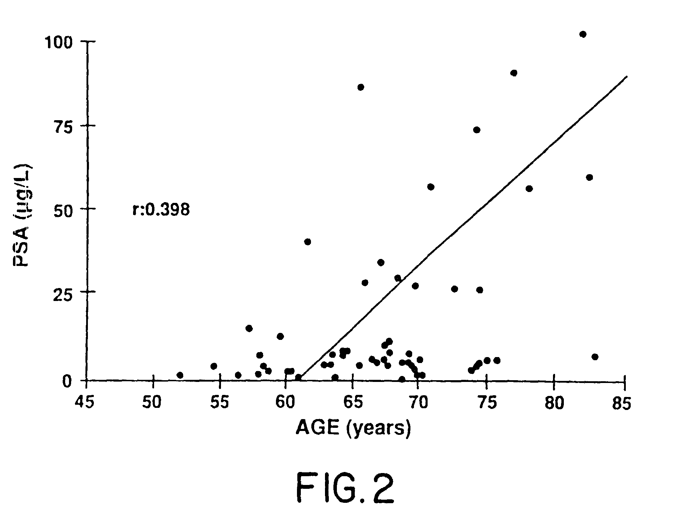

Figure 1 shows that the distribution of serum PSA concentrations as a function of age displays a slight tendency to an increase with age with a very low coefficient of correlation (r: 0.184, N.S.). Although an increase in the volume of the prostate caused by BPH has been found to cause an elevation in serum PSA (Stamey et al., J. Urol. 141: 1076-1083, 1989), data of Figure 1 do not support the use of an age-related correction factor to take into account the presumed increasing contribution of BPH to the serum PSA levels with increasing age. It is of interest to see in Figure 2, that there is, however, a significant correlation between serum PSA levels in men diagnosed as having prostate cancer and age. However, as can also be seen in Fig. 2 and more clearly indicated in Table 2, at ages ranging from 50 to 60 years, 2 cancers were detected in men having serum PSA values up to 3. 0 ng/ml while in the ages ranging between 60 and 70 and 70 to 85, the numbers of men having cancer with normal serum PSA values were 6 and 3, respectively. Similarly, cancer was found at serum PSA levels ranging from 3.1 to 6.0 ng/ml in 4, 9 and 4 men aged between 50 and 60, 60 and 70 and 70 to 85 years, respectively, thus clearly indicating that an increase of the threshold serum PSA value with age would result in a marked decrease in the sensitivity of the prescreening test.

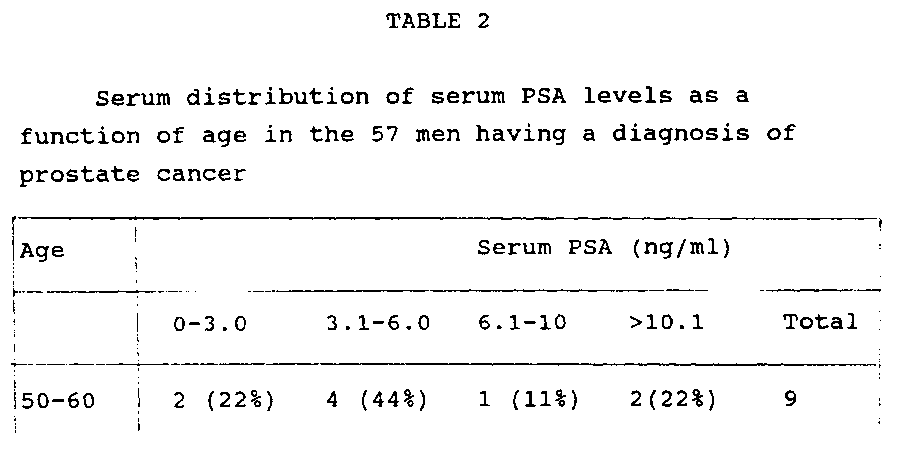

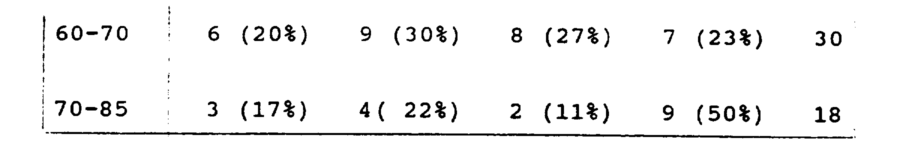

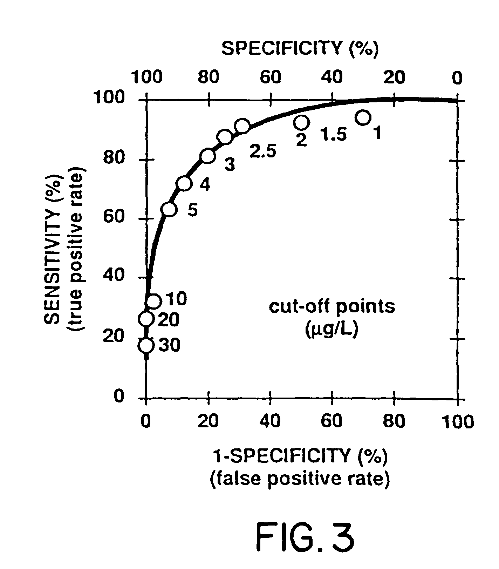

In order to optimize the threshold value of normal serum PSA, the sensitivity and specificity of various cut-off values are presented in Table 3. It can be seen that at a threshold value of 3.0 ng/ml, the sensitivity and specificity are 80.7% and 84.6%, respectively. The relationship between sensitivity and specificity of the PSA test can be best expressed by the receiver operating characteristic (ROC) curve (Fig. 3). Using this correlation, the cut-off point at 3.0 ng/ml indicates optimal values for both sensitivity and specificity.

### TABLE 3

Sensitivity and specificity calculated for different threshold values of serum PSA and the 95% confidence intervals (C.I.)

| PSA threshold (ng/ml) | Sensitivity ± C. I. | Specificity ± C. I. |
|---|---|---|
| 1.0 | 92.9 ± 6.7% | 39.4 ± 3.0% |
| 1.5 | 89.3 ± -8.9% | 58.9 ± 3.1% |
| 2 | 89.3 ± 8.9% | 73.0 ± 3.2% |
| 2.5 | 85.7 ± 9.1% | 79.3 ± 2.6% |
| 3 | 80.7 ± 10.2% | 84.6 ± -2.3% |
| 4 | 71.4 ± 11.7% | 91.1 ± 1.8% |
| 5 | 62.5 ± 12.6% | 93.6 ± 1.6% |
| 10 | 32.1 ± 12.1% | 98.2 ± 0.8% |
| 20 | 26.8 ± 11.5% | 99.8 ± 0.3% |
| 30 | 17.9 ± 9.9% | 99.9 ± 0.2% |

In fact, the aim of a prescreening test should be a high probability of having prostate cancer when serum PSA is abnormal while a minimum of cancers should be present when serum PSA is normal. Positive and negative predictive values depend not only on sensitivity and specificity but also on prevalence of the disease in the target population. At a threshold value of 3.0 ng/ml, the positive predictive value of the test is 24.1% while its negative predictive value is 98.6% (Table 4).

### TABLE 4

Positive (+PV) and negative (-PV) predictive values calculated for different serum PSA thresholdconcentrations

| PSA threshold (ng/ml) | + PV (%) | -PV (%) |
|---|---|---|
| 1.0 | 8.4 | 98.9 |
| 1.5 | 11.5 | 98.9 |
| 2 | 16.1 | 99.1 |
| 2.5 | 19.8 | 98.9 |
| 3 | 24.1 | 98.6 |
| 4 | 32.5 | 98.2 |
| 5 | 36.8 | 97.7 |
| 10 | 51.4 | 96.0 |
| 20 | 88.2 | 95.8 |
| 30 | 90.0 | 95.3 |

Table 5 shows the predictive values estimated from the 2 x 2 table (Gale and Gambino, beyond normality. In: The predictive value and Efficiency of Medical Diagnosis, John Wiley and sons, New York, 1975, 237p). for the population screend in our study and for teh general population calculated accordining to the Bayes theorem (Galen and Gambino, Beyond normality. In: The predictive value and Efficiency of Medical Diagnosis, John Wiley and sons, New York, 1975, 237pp).

Positive and negative predictive values calculated for our randomized screened population and the general population (prevalence = 5%) when the upper normal limit of PsA test was fixed at 3.0 ng/ml.

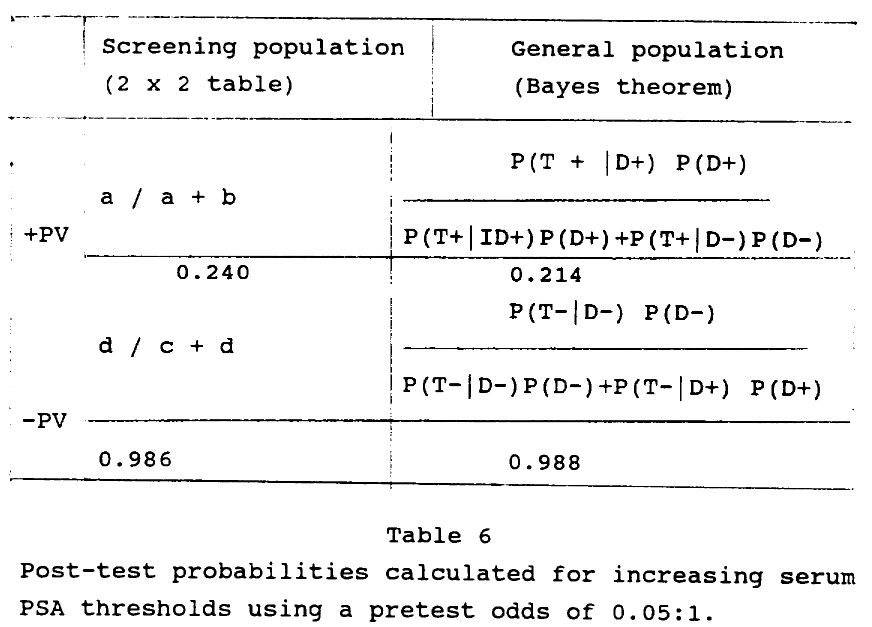

| PSA threshold (ng/ml) | Probability of prostate cancer |
|---|---|
| 1.0 | 7.5% |
| 1.5 | 10.2% |
| 2.0 | 14.5% |
| 2.5 | 17.9% |
| 3.0 | 21.6% |
| 4.0 | 29.7% |
| 5.0 | 33.9% |
| 10 | 48.4% |
| 20 | 87.6% |
| 30 | 90.4% |

The similarity of predictive values observed between the screened and the general population reflects the excellent representativity of our sample for the general population. The prevalence seen in our cohort was 5.69% compared with 5% reported for the general population (Torp-Pedersen and Lee, 1990).

Likelihood ratios offer an alternative method of describing sensitivity and specificity and can be used to calculate the probability of prostate cancer for a given serum PSA concentration. The diagnostic power of the likelihood-ratio approach is best illustrated in Table 6, where post-test probability has been calculated as described by Fletcher et al. (Fletcher et al., Clinical Epidemiology, The Essentials, 2nd Edition, 1988, 240 pp) using a pretest odds of 0. 05: 1. It can be seen that at a cut-off value of 3.0 jig/L, the probability of prostate cancer is 21.6%, a value similar to the positive predictive value calculated in Table 4.

Since only 54% (41 of 76) of patients originally classified as having clinical stage A disease had organ-confirmed disease following histopathological examination of the surgical specimen (Paulson, in Proceedings of the 5th Int. Symp. on Transrectal Ultrasound in the Diagnosis and Management of Prostate Cancer, Chicago, pp. 34-50, 1990), early diagnosis and treatment become particularly important if the possibility of a cure can be offered to the patient. Moreover, 11 of 18 (61%) of preoperative stage A patients were upstaged to A₂ or C following histopathological examination of the surgical specimen. Even using digital rectal examination, a low sensitivity technique, it has been found that routine screening in a given population increased the percentage of patients with clinically curable carcinoma of the prostate (Thompson et al., J. Urol. 137, 424, 1987).

## Claims

1. A method for routine prescreening for prostate cancer in a male person, said method comprising the steps of:
(A) obtaining a serum sample from said person; and
(B) determining whether a concentration of prostatic specific antigen in said sample exceeds a predetermined threshold value, said threshold value being between 2.0 and 3.9 nanograms of prostatic specific antigen per ml of serum in said sample, when measured by TANDOM R™ assay (available from Hybritech), a two-site monoclonal immunometric (solid-phase) assay using pooled female sera as a zero control.

2. The method of claim 1, wherein said threshold value is between 2.5 and 3.5 nanograms of prostatic specific antigen per ml of serum in said sample.

3. The method of claim 1, wherein said threshold value is between 2.0 and 2.5 nanograms of prostatic specific antigen per ml of serum in said sample.

4. The method of claim 1, wherein said threshold value is 3.0 nanograms of prostatic specific antigen per ml of serum in said sample.

5. The method of claim 1, wherein said threshold value is between 3.0 and 3.9 nanograms of prostatic specific antigen per ml of serum in said sample.

6. A kit for routine prescreening of male persons for prostate cancer, said kit comprising:
(A) a means for taking and receiving a blood sample from said person; and
(B) a means for determining whether said sample includes a concentration of prostatic specific antigen which is above a predetermined threshold value, said means performing the functions of measuring said concentration of prostatic specific antigen, comparing measured concentration to said threshold value, and reporting a positive result when said threshold is exceeded and a negative result when said threshold is not exceeded, wherein said threshold value is set between 2.0 and 3.9 nanograms of prostatic specific antigen per ml of serum in said sample when measured by TANDOM R™ (available from Hybritech), a two-site monoclonal immunometric (solid-phase) assay using pooled female sera as a zero control.

7. The kit of claim 6, wherein said threshold value is between 2.5 and 3.5 nanograms of prostatic specific antigen per ml of serum in said sample.

8. The kit of claim 6, wherein said threshold value is between 2.0 and 2.5 nanograms of prostatic specific antigen per ml of serum in said sample.

9. The kit of claim 6, wherein said threshold value is 3.0 nanograms of prostatic specific antigen per ml of serum in said sample.

10. The kit of claim 6, wherein said threshold value is between 3.0 and 3.9 nanograms of prostatic specific antigen per ml of serum in said sample.

## Patentansprüche

1. Verfahren zur Routine-Vorprüfung auf Prostatakrebs bei einer männlichen Person, wobei das Verfahren folgende Schritte umfaßt:
(A) Gewinnen einer Serumprobe von der Person; und
(B) Bestimmen, ob eine Konzentration an prostataspezifischem Antigen in der Probe einen vorgegebenen Schwellwert übersteigt, wobei der Schwellwert zwischen 2,0 Nanogramm und 3,9 Nanogramm des prostataspezifischen Antigens pro ml Serum in der Probe liegt, wenn mit einer TANDOM R^{™}-Analyse (erhältlich von Hybritech), einer monoklonalen immunometrischen (Festphasen-)Analyse mit zwei aktiven Stellen, unter Verwendung von weiblichen Pool-Seren als Null-Kontrolle gemessen wird.

2. Verfahren nach Anspruch 1, wobei der Schwellwert zwischen 2,5 Nanogramm und 3,5 Nanogramm des prostataspezifischen Antigens pro ml Serum in der Probe liegt.

3. Verfahren nach Anspruch 1, wobei der Schwellwert zwischen 2,0 Nanogramm und 2,5 Nanogramm des prostataspezifischen Antigens pro ml Serum in der Probe liegt.

4. Verfahren nach Anspruch 1, wobei der Schwellwert 3,0 Nanogramm des prostataspezifischen Antigens pro ml Serum in der Probe beträgt.

5. Verfahren nach Anspruch 1, wobei der Schwellwert zwischen 3,0 Nanogramm und 3,9 Nanogramm des prostataspezifischen Antigens pro ml Serum in der Probe liegt.

6. Ausrüstung zur Routine-Vorprüfung von männlichen Personen auf Prostatakrebs, wobei die Ausrüstung beinhaltet:
(A) ein Mittel zum Entnehmen und Erhalten einer Blutprobe von der Person; und
(B) ein Mittel zum Bestimmen, ob die Probe eine Konzentration des prostataspezifischen Antigens beinhaltet, die oberhalb eines vorgegebenen Schwellwerts liegt wobei das Mittel die Funktionen des Messens der Konzentration des prostataspezifischen Prostata-Antigens, des Vergleichens der gemessenen Konzentration mit dem Schwellwert und des Berichtens eines positiven Resultats, wenn der Schwellwert überschritten wird, und eines negativen Resultats ausführt, wenn der Schwellwert nicht überschritten wird, wobei der Schwellwert zwischen 2,0 Nanogramm und 3,9 Nanogramm des prostataspezifischen Antigens pro ml Serum in der Probe liegend festgelegt ist, wenn mit einer TANDOM R™-Analyse (erhältlich von Hybritech), einer monoklonalen immunometrischen (Festphasen-)Analyse mit zwei aktiven Stellen, unter Verwendung von weiblichen Pool-Seren als Null-Kontrolle gemessen wird.

7. Ausrüstung nach Anspruch 6, wobei der Schwellwert zwischen 2,5 Nanogramm und 3,5 Nanogramm des prostataspezifischen Antigens pro ml Serum in der Probe liegt.

8. Ausrüstung nach Anspruch 6, wobei der Schwellwert zwischen 2,0 Nanogramm und 2,5 Nanogramm des prostataspezifischen Antigens pro ml Serum in der Probe liegt.

9. Ausrüstung nach Anspruch 6, wobei der Schwellwert 3,0 Nanogramm des prostataspezifischen Antigens pro ml Serum in der Probe beträgt.

10. Ausrüstung nach Anspruch 6, wobei der Schwellwert zwischen 3,0 Nanogramm und 3,9 Nanogramm des prostataspezifischen Antigens pro ml Serum in der Probe liegt.

## Revendications

1. Méthode de criblage préalable de routine pour le cancer de la prostate dans une personne de sexe masculin, ladite méthode comprenant les étapes :
A/ obtenir un échantillon de sérum de ladite personne;
B/ déterminer si une concentration en antigène spécifique de la prostate dans ledit échantillon dépasse un seuil prédéterminé; ledit seuil étant une vapeur comprise entre 2,0 et 3,9 nanogrammes d'antigène spécifique de la prostate par ml de sérum dans ledit échantillon, lorsque mesuré à l'aide de l'essai TANDOM R^{™} (disponible auprès d'Hybritech), un essai immunométrique monoclonal à deux sites (en phase solide) utilisant des serum féminins poolés comme contrôle zéro.

2. Méthode selon la revendication 1 où ledit seuil est compris entre 2,5 et 3,5 nanogrammes d'antigène spécifique de la prostate par ml de sérum dans ledit échantillon.

3. Méthode selon la revendication 1, où ledit seuil est compris entre 2,0 et 2,5 nanogrammes d'antigène spécifique de la prostate par ml de sérum dans ledit échantillon.

4. Méthode selon la revendication 1, où ledit seuil est 3,0 nanogrammes d'antigène spécifique de la prostate par ml de sérum dans ledit échantillon.

5. Méthode selon la revendication 1, où ledit seuil est compris entre 3,0 et 3,9 nanogrammes d'antigène spécifique de la prostate par ml de sérum dans ledit échantillon.

6. Trousse pour un criblage préalable de routine de personnes de sexe mâle pour le cancer de la prostate, ladite trousse comprenant :
A/ un moyen pour prélever et pour recueillir un échantillon de sang de ladite personne;
B/ un moyen pour déterminer si ledit échantillon comprend une concentration en antigène spécifique de la prostate qui supéieure à un seuil prédéterminé, le dit moyen performant les fonctions de mesure de la dite concentration en antigène spécifique de la prostate par comparaison de la concentration mesurée audit seuil, et en indiquant un résultat positif lorsque ledit seuil est dépassé et un résultat négatif lorsque ledit n'est pas dépassé, où ladite valeur de seuil est fixée entre 2,0 et 3,9 nanogrammes d'antigène spécifique de la prostate par ml de sérum dans ledit échantillon lorsque mesurée à l'aide du TANDOM RTM (disponible auprès d'Hybritech), un essai immunométrique (en phase solide) monoclonal à deux sites utilisant des serum féminins poolés comme contrôle zéro.

7. Trousse de la revendication 6, où ledit seuil est compris entre 2,5 et 3,5 nanogrammes d'antigène spécifique de la prostate par ml de sérum dans ledit échantillon.

8. Trousse de la revendication 6, où ledit seuil est compris entre 2,0 et 2,5 nanogrammes d'antigène spécifique de la prostate par ml de sérum dans ledit échantillon.

9. Trousse de la revendication 6, où ledit seuil est 3,0 nanogrammes d'antigène spécifique de la prostate par ml de sérum dans ledit échantillon.

10. Trousse de la revendication 6, où ledit seuil est compris entre 3,0 et 3,9 nanogrammes d'antigène spécifique de la prostate par ml de sérum dans ledit échantillon.
